# EUROPEAN PATENT APPLICATION

(11) **EP 4 729 038 A1**
(43) Date of publication of application: **22.04.2026**
(21) Application number: 24868125.6
(22) Date of filing: 05.09.2024
(51) Int. Cl.: A61F 13/56, A61F 13/15, A61F 13/496

(54) **ABSORBENT ARTICLE AND PACKAGE OF ABSORBENT ARTICLE**

(30) Priority: 19.09.2023 JP 2023151727
(71) Applicant: Unicharm Corporation, Ehime 799-0111 (JP)
(72) Inventor: MURAKAMI, Hiroko, Kanonji-shi, Kagawa 769-1602 (JP); SAITO, Kyota, Kanonji-shi, Kagawa 769-1602 (JP); DINH, Nhu, Chien, Bac Ninh Province (VN)
(74) Representative: Dolleymores
(86) International application number: PCT/JP2024/031837
(87) International publication number: WO 2025/063047

(57) **Abstract**

An absorbent article (1) has an annular waistline part provided with an abdomen-side waistline part (20) and a back-side waistline part (30), and an absorbent body (10). The absorbent article (1) has a fastening part (5). The base end part of the fastening part (5) is fixed to the waistline parts (20, 30) by a fixing part (F1). The tip part of the fastening part (5) has an engagement part (51) that detachably engages with a non-skin-side surface of the waistline parts (20, 30) has a cutting guide part (40) that guides cutting of the waistline parts (20, 30) in the up-down direction, and has, in the cutting guide part (40), a partial cut part (41) in which parts of the waistline parts (20, 30) are cut in the up-down direction.

## Description

### FIELD

The present invention relates to an absorbent article and an absorbent article package.

### BACKGROUND

A conventionally known absorbent article includes a leg opening on one side in the left-right direction, and is configured such that a leg opening is formable also on the other side by engaging a fastening portion, which is provided on the other side in the left-right direction, with the waist portion. Such an absorbent article can be worn by first passing one leg of the wearer through the leg opening on one side and then pulling up the absorbent article to the wearer's crotch section, and thereafter, engaging the fastening portion on the other side. For example, Patent Document 1 discloses a disposable diaper wherein one side of the left and right lateral portions of the waist portion (hip portion) is joined together to form a leg hole, whereas the other side is not joined but is provided with a tape fastener capable of forming a leg hole in the non-joined waist portion.

### [CITATION LIST]

### [PATENT LITERATURE]

[Patent Document 1] Japanese Examined Utility Model Application Publication No. H07-17304

### SUMMARY

### [TECHNICAL PROBLEM]

In the absorbent article of Patent Document 1, one side of the left and right lateral portions of the waist portion is not joined, and thus, the waist portion is not annular. This may make it difficult to control the conveyance of the waist portion on a manufacturing line, thereby inhibiting stable mass production of absorbent articles. To address this, an absorbent article including an annular waist portion and a fastening portion has been proposed. When this absorbent article is used, a user first cuts open the annular waist portion in the up-down direction and then re-connects the waist portion with the fastening portion so as to fit the wearer's size, thereby enabling attachment of the absorbent article. There are, however, cases where the absorbent article is not worn properly-for example, where it is difficult for the user to understand that the annular waist portion needs to be cut before attachment, or to recognize the cutting position of the waist portion.

The present invention was achieved in light of conventional problems such as those described above, and an objective of the invention is to provide an absorbent article in which it is easy to recognize that the annular waist portion is to be cut before attachment, and to recognize the cutting position of the waist portion.

### [SOLUTION TO PROBLEM]

A main aspect of the invention for achieving the above-described objective is an absorbent article having an up-down direction, a left-right direction, and a front-back direction intersecting with one another, absorbent article including: an annular waist portion including a front waist portion and a back waist portion; an absorbent main body; and a fastening portion, a base end portion of the fastening portion being fixed to the waist portion by a fixing portion, and a leading end portion of the fastening portion including an engaging portion configured to detachably engage with a non-skin-side face of the waist portion, the absorbent article including a cutting-guide portion configured to guide cutting of the waist portion in the up-down direction, and the absorbent article including, in the cutting-guide portion, a partially-cut portion in which a portion of the waist portion in the up-down direction has been cut.

Other features of the present invention will become clear through the description of the present specification and the accompanying drawings.

### [ADVANTAGEOUS EFFECT OF THE INVENTION]

According to the present invention, it is possible to provide an absorbent article in which it is easy to recognize that the annular waist portion is to be cut before attachment, and to recognize the cutting position of the waist portion.

### BRIEF DESCRIPTION OF DRAWINGS

FIG. 1 is a perspective view of disposable diaper 1 in a worn state.
FIG. 2 is a plan view of diaper 1 in an unfolded and stretched state as viewed from the side of a non-skin-side face.
FIG. 3 is an enlarged view of region X in FIG. 2.
FIG. 4 is an enlarged view of region X in FIG. 2.
FIG. 5 illustrates separation of front waist portion 20 and formation of a waist opening.
FIG. 6 illustrates actions for cutting front waist portion 20.
FIG. 7 illustrates partially-cut portion 41.
FIG. 8 illustrates a modified example of partially-cut portion 41.
FIG. 9 illustrates modified examples of cutting-guide portion 40 and partially-cut portion 41.
FIG. 10 illustrates a modified example of partially-cut portion 41.
FIG. 11 illustrates modified examples of diaper 1.
FIG. 12 is a diagram illustrating adhesive HMA joining skin-side sheet 21 and non-skin-side sheet 22.
FIG. 13 is a diagram illustrating a modified example of cutting-guide portion 40.
FIG. 14 is a diagram illustrating package 60 of diapers 1.

### DESCRIPTION OF EMBODIMENTS

At least the following matters are disclosed in the description of this specification and the attached drawings.

Aspect 1:
an absorbent article having an up-down direction, a left-right direction, and a front-back direction intersecting with one another, absorbent article including: an annular waist portion including a front waist portion and a back waist portion; an absorbent main body; and a fastening portion, a base end portion of the fastening portion being fixed to the waist portion by a fixing portion, and a leading end portion of the fastening portion including an engaging portion configured to detachably engage with a non-skin-side face of the waist portion, the absorbent article including a cutting-guide portion configured to guide cutting of the waist portion in the up-down direction, and the absorbent article including, in the cutting-guide portion, a partially-cut portion in which a portion of the waist portion in the up-down direction has been cut.

According to Aspect 1, a user having visually recognized the partially-cut portion can easily recognize that the waist portion is to be cut before attachment and can also easily recognize the cutting position of the waist portion. Thus, the user can cut the waist portion along the cutting-guide portion and can easily perform actions for attaching the absorbent article.

### Aspect 2:

The absorbent article according to Aspect 1, wherein
at least a portion of the cutting-guide portion is located, in the left-right direction, between the fixing portion and an end of the engaging portion on the leading end portion side of the fastening portion, or the cutting-guide portion is the fixing portion.

According to Aspect 2, the fastening portion, which is fixed to the waist portion by the engaging portion and the fixing portion, can inhibit stretching of the waist portion in the left-right direction. Thus, even when the waist portion has been cut at the partially-cut portion, the partially-cut portion can be inhibited from opening in the left-right direction, thereby inhibiting unnecessary cutting of the waist portion at the cutting-guide portion.

### Aspect 3:

The absorbent article according to Aspect 2, wherein
in a stretched state of the waist portion, at least a portion of the partially-cut portion overlaps the engaging portion in the up-down direction.

According to Aspect 3, the partially-cut portion is located on an extension, in the left-right direction, of the engaging portion, and thus, the partially-cut portion can be inhibited from opening in the left-right direction. Further, when a user lifts up the fastening portion, tensile force is likely to concentrate on the engaging portion and be transmitted to the partially-cut portion, thereby allowing the waist portion to be cut more easily, starting from the partially-cut portion.

### Aspect 4:

The absorbent article according to Aspect 2, wherein
in a stretched state of the waist portion, the partially-cut portion does not overlap the engaging portion in the up-down direction.

According to Aspect 4, the partially-cut portion is provided in a region of the waist portion where the restriction of movement by the fastening portion is small. This makes the partially-cut portion more easily visible, enabling the user to recognize the partially-cut portion more easily.

### Aspect 5:

The absorbent article according to any one of Aspects 2 to 4, wherein
at least a portion of the partially-cut portion overlaps the fastening portion in the front-back direction.

According to Aspect 5, by covering the partially-cut portion with the fastening portion, unnecessary cutting of the waist portion starting from the partially-cut portion can be inhibited. Further, the partially-cut portion is visually recognized when a user lifts up the fastening portion, and the partially-cut portion can thus be readily recognized as the cutting position of the waist portion.

### Aspect 6:

The absorbent article according to any one of Aspects 1 to 5, wherein
the waist portion includes, in the cutting-guide portion, a non-cut portion in which the waist portion has not been cut, and in a stretched state of the waist portion, a length of the partially-cut portion in the up-down direction is different from a length of the non-cut portion in the up-down direction.

According to Aspect 6, in cases where the length of the partially-cut portion is longer, the partially-cut portion becomes easier for the user to recognize, and the user can cut the waist portion easily. In cases where the non-cut portion is longer, unnecessary cutting of the waist portion starting from the partially-cut portion can be inhibited.

### Aspect 7:

The absorbent article according to any one of Aspects 1 to 6, wherein
the waist portion is formed by a plurality of sheet members, each of the plurality of sheet members is joined by an adhesive with the sheet member adjacent thereto in a thickness direction of the waist portion, and the adhesive reaches at least an end of the partially-cut portion located on one side in the left-right direction.

According to Aspect 7, the sheets can be inhibited from opening at the edge, in the left-right direction, of the partially-cut portion, and the visual recognizability of the partially-cut portion can be improved.

### Aspect 8:

The absorbent article according to any one of Aspects 1 to 7, wherein
the waist portion is formed by a plurality of sheet members, each of the plurality of sheet members is joined by an adhesive with the sheet member adjacent thereto in a thickness direction of the waist portion, and the adhesive is located so as to extend across the cutting-guide portion in the left-right direction.

According to Aspect 8, unnecessary cutting of the waist portion starting from the partially-cut portion can be inhibited at the intersection portion between the cutting-guide portion and the adhesive.

### Aspect 9:

The absorbent article according to any one of Aspects 1 to 8, wherein
the cutting-guide portion includes an upper inclined portion that is inclined upwardly from a central portion in the up-down direction and toward the base end portion side of the fastening portion, and a lower inclined portion that is inclined downwardly from the central portion and toward the base end portion side of the fastening portion, and the partially-cut portion is located so as to extend across an intersection point between the upper inclined portion and the lower inclined portion.

According to Aspect 9, since the waist portion has already been cut by the partially-cut portion at the intersection point between the upper inclined portion and the lower inclined portion serving as the cutting start point, when a user pulls the fastening portion and the waist portion, the waist portion can be readily cut along the cutting-guide portion from the partially-cut portion.

### Aspect 10:

The absorbent article according to any one of Aspects 1 to 9, wherein
the fastening portion includes a grip portion, the grip portion is a portion of the fastening portion disposed further toward the leading end portion side than the engaging portion, and in a stretched state of the waist portion, at least a portion of the partially-cut portion overlaps the grip portion in the up-down direction.

According to Aspect 10, when a user grips the grip portion and pulls the fastening portion, the tensile force can be readily applied to the partially-cut portion which is located on an extension of the grip portion in the left-right direction. Thus, the waist portion can be readily cut along the cutting-guide portion from the partially-cut portion.

### Aspect 11:

The absorbent article according to any one of Aspects 2 to 10, wherein
a color of a skin-side face of the fastening portion is different from a color of the non-skin-side face of the waist portion.

According to Aspect 11, when a user lifts up the fastening portion, the color of the fastening portion can be visually recognized from between the partially-cut portion, making the partially-cut portion stand out. Thus, the partially-cut portion can be readily recognized by the user.

### Aspect 12:

The absorbent article according to any one of Aspects 1 to 11, wherein
the waist portion is formed by one or more sheet members, and a tensile strength of the one or more sheet members in the left-right direction is higher than a tensile strength of the one or more sheet members in the up-down direction.

According to Aspect 12, the waist portion can be inhibited from stretching in the left-right direction, and the partially-cut portion can be inhibited from opening toward the left and right. Thus, unnecessary cutting of the waist portion starting from the partially-cut portion can be inhibited.

### Aspect 13:

The absorbent article according to any one of Aspects 1 to 12, wherein
the waist portion is formed by one or more sheet members, the fastening portion is formed by one or more fastening-portion sheet members, and a maximum value of a total grammage of the one or more sheet members is smaller than a minimum value of a total grammage of the one or more fastening-portion sheet members.

According to Aspect 13, since the total grammage of the sheet member(s) constituting the waist portion is small, the waist portion can be cut easily, and the partially-cut portion can be formed easily. Conversely, since the total grammage of the fastening-portion sheet member(s) constituting the fastening portion is large, the fixed state of the fastening portion to the waist portion can be maintained easily.

Aspect 14:

The absorbent article according to any one of Aspects 2 to 13, wherein
on a side further toward the base end portion of the fastening portion than the cutting-guide portion in the left-right direction, the absorbent article includes a joining portion that joins the fastening portion and the waist portion, and in a stretched state of the waist portion, at least a portion of the partially-cut portion overlaps the joining portion in the up-down direction.

According to Aspect 14, fixing of the fastening portion to the waist portion is reinforced by the joining portion, thereby further inhibiting the waist portion from stretching in the left-right direction. Thus, the partially-cut portion can be inhibited from opening in the left-right direction, thereby inhibiting unnecessary cutting of the waist portion starting from the partially-cut portion.

### Aspect 15:

The absorbent article according to any one of Aspects 1 to 14, wherein
the partially-cut portion is located further inward than an end of the waist portion in the up-down direction, and a plurality of the partially-cut portions are lined up in the up-down direction.

According to Aspect 15, a plurality of through holes in the waist portion are lined up in the partially-cut portion, and thus, the partially-cut portion is visually recognized as a perforated line. Thus, a user can readily recognize that the waist portion is to be cut at the partially-cut portion.

### Aspect 16:

The plurality of absorbent articles according to any one of Aspects 1 to 15, wherein
the absorbent article includes a non-cutting-guide portion at least on one side, in the up-down direction, of the cutting-guide portion.

According to Aspect 16, unnecessary cutting of the waist portion starting from the partially-cut portion can be inhibited at the non-cutting-guide portion. Further, by adjusting the arrangement of the non-cutting-guide portion, it becomes easier to form a desired partially-cut portion.

### Aspect 17:

The plurality of absorbent articles according to Aspect 16, wherein
the absorbent article includes a welded portion in which the waist portion and the fastening portion are welded, the welded portion is disposed adjacent to the cutting-guide portion on the base end portion side of the fastening portion in the left-right direction, and in a stretched state of the waist portion, at least a portion of the non-cutting-guide portion overlaps the welded portion in the up-down direction.

According to Aspect 17, the welded portion can assist the cutting of the waist portion at the non-cutting-guide portion. Thus, a user can smoothly cut the waist portion along the cutting-guide portion.

Aspect 18: An absorbent article package including: a plurality of the absorbent articles according to claim 1; and a packaging member that packages the plurality of absorbent articles, the plurality of absorbent articles including a first absorbent article and a second absorbent article, and a relative position of the partially-cut portion relative to the cutting-guide portion of the first absorbent article being different from a relative position of the partially-cut portion relative to the cutting-guide portion of the second absorbent article.

According to Aspect 18, a user who has visually recognized a plurality of different positions of the partially-cut portions relative to the cutting-guide portion will be able to more easily recognize the overall shape of the cutting-guide portion. Thus, the user can be more aware of cutting the waist portion along the cutting-guide portion, and can readily cut the waist portion.

### EMBODIMENTS:

An embodiment of an absorbent article according to the present invention will be described below, taking an underpants-shaped disposable diaper for infant/toddler use as an example. Note, however, that the absorbent article of the present invention is not limited to disposable diapers for infant/toddler use, and is applicable to disposable diapers for adult use, sanitary underpants, etc.

### Configuration of Disposable Diaper 1:

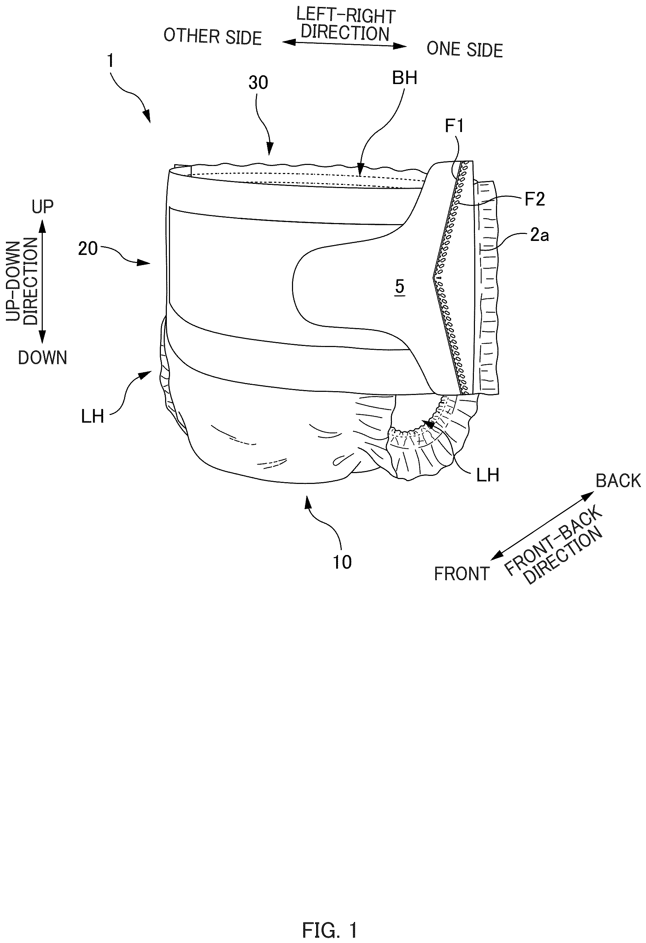

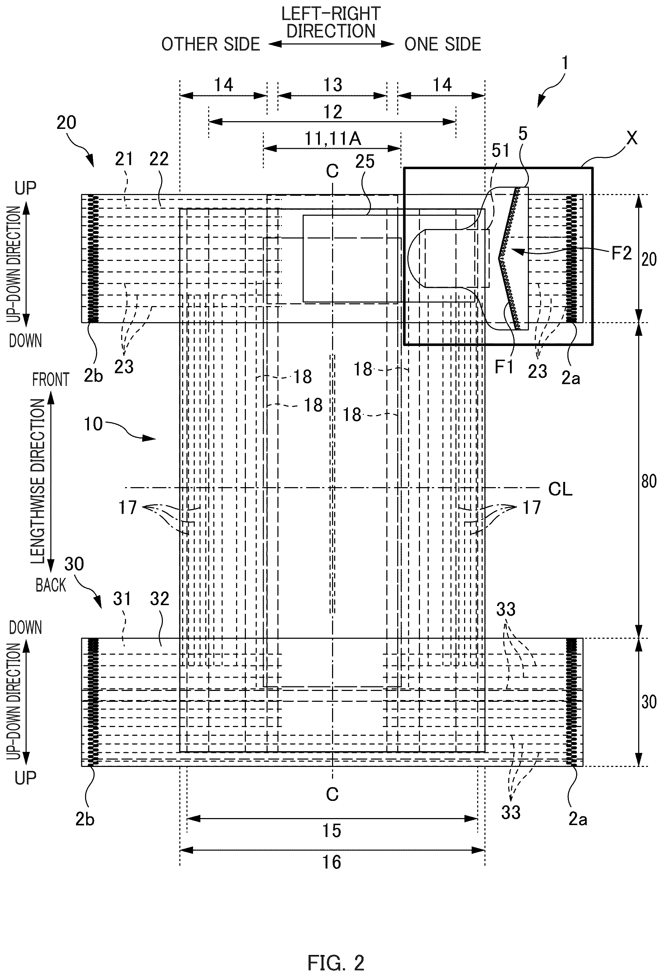

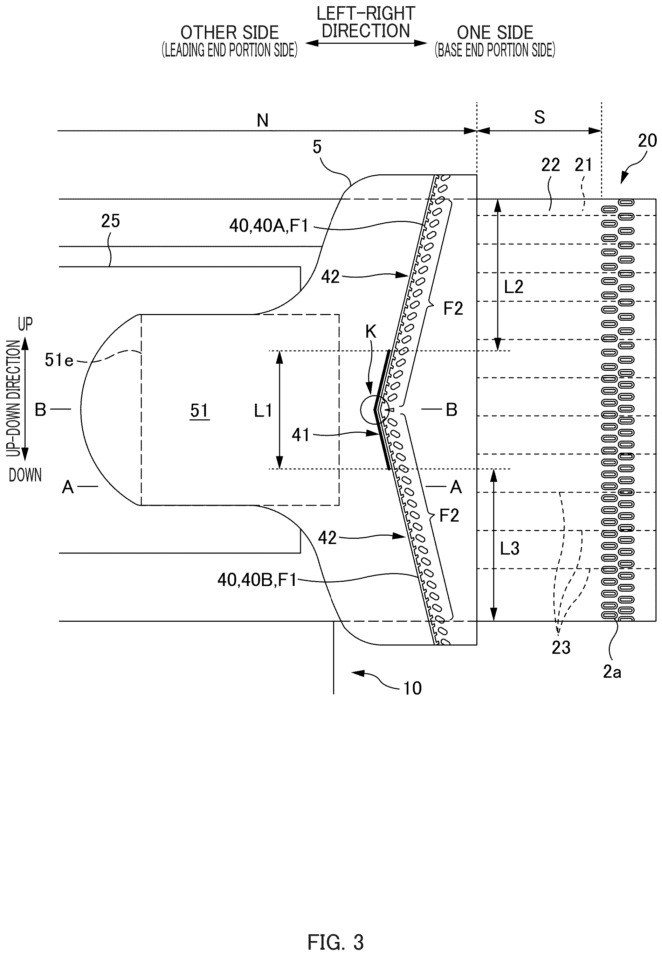

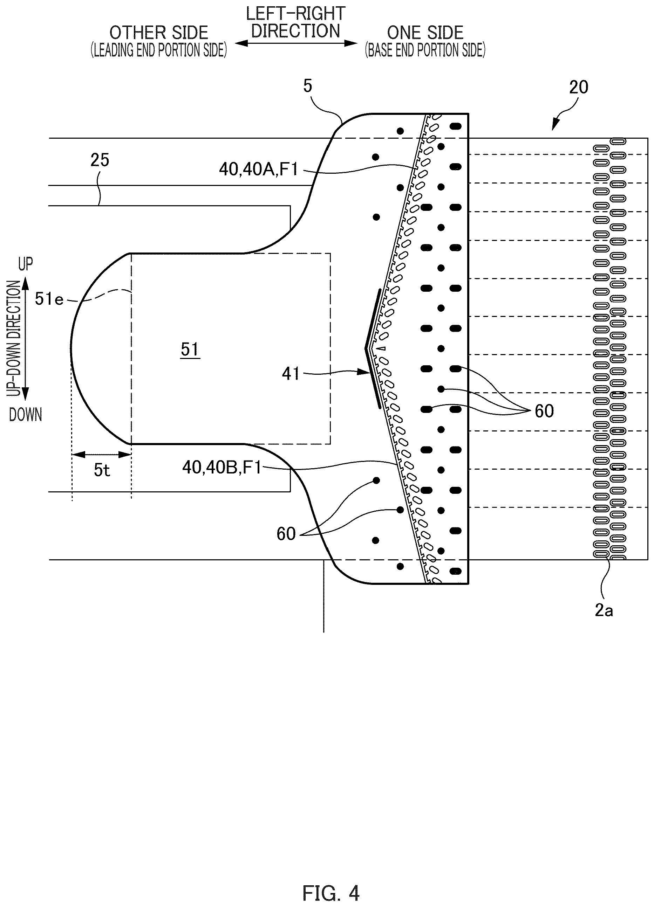

FIG. 1 is a perspective view of a disposable diaper 1 (hereinbelow "diaper") in a worn state, and is a perspective view as viewed from a "one side" in the left-right direction. FIG. 2 is a plan view of the diaper 1 in an unfolded and stretched state as viewed from the side of a non-skin-side face. In FIG. 2, central line C-C is a central line in the left-right direction in a stretched state. FIGS. 3 and 4 are enlarged views of region X in FIG. 2.

"Natural state" refers to a state in which a diaper 1 has been left to stand for a predetermined time. For example, a diaper 1 in a product state is taken out from a package, and the diaper's back waist portion 30 is pulled toward both outer sides in the left-right direction to bring the waist portion 20, 30 into a "stretched state". This stretched state is maintained for 15 seconds, and then the tension on the diaper 1 is released and the diaper is laid on a flat surface such as a desk. A state after passage of 5 minutes in this laid-flat state is referred to as "natural state".

"Unfolded state" refers to a state in which both side portions of an underpants-shaped diaper 1-i.e., side joining portions 2a, 2b formed between both lateral end portions of a front waist portion 20 and both lateral end portions of a back waist portion 30-have been separated and the entire diaper 1 has been opened up and spread out in a planar state. "Stretched state" refers to a state in which stretchable/contractible members provided in the diaper 1 have been stretched to an extent that the wrinkles in the diaper 1 are no longer visually recognizable. More specifically, it refers to a state in which the diaper has been stretched out to an extent that the size of each member (e.g., front waist portion 20 described below) constituting the diaper 1 matches, or is close to, the size of that member alone.

In the underpants-shaped state illustrated in FIG. 1, the diaper 1 has an up-down direction, a left-right direction, and a front-back direction intersecting with (or orthogonal to) one another, and has a waist opening BH and a pair of leg openings LH, LH. In the up-down direction, the side of the waist opening BH is defined as "up", whereas the crotch side is defined as "down". In the front-back direction, the wearer's abdomen side is defined as "front side", whereas the wearer's rear side is defined as "back side". The direction in which constituent materials of the diaper 1 are layered is defined as "thickness direction", wherein the side, in the thickness direction, that comes into contact with the wearer is defined as "skin side", whereas the side opposite from the skin side is defined as "non-skin side". It should be noted that the thickness direction is also the direction along the front-back direction in the natural state.

The diaper 1 is a so-called three-piece-type diaper, and includes an annular waist portion including a front waist portion 20 and a back waist portion 30, and an absorbent main body 10. The front waist portion 20 is for covering the wearer's abdomen side, and the back waist portion 30 is for covering the wearer's rear side. The diaper 1 also includes a fastening portion 5.

In the diaper 1 in its unfolded state as illustrated in FIG. 2, the front waist portion 20 and the back waist portion 30 are disposed such that their lengthwise direction is oriented along the left-right direction of the diaper 1. Further, a crotch portion 80 is formed by: disposing the absorbent main body 10's end portion on one side in the absorbent main body's lengthwise direction at the central portion, in the left-right direction, of the front waist portion 20; and disposing the absorbent main body 10's end portion on the other side in the absorbent main body's lengthwise direction at the central portion, in the left-right direction, of the back waist portion 30. The absorbent main body 10 is disposed further toward the skin side than the front waist portion 20 and the back waist portion 30.

The diaper 1 in its unfolded state, as illustrated in FIG. 2, is folded in two at substantially the center CL, in the lengthwise direction, of the absorbent main body 10 in a state where the lengthwise direction of the absorbent main body 10 is oriented along the up-down direction of the diaper 1, and a pair of side joining portions 2a, 2b is formed by joining both lateral end portions, in the diaper 1's left-right direction, of the front waist portion 20 with both lateral end portions of the back waist portion 30 by thermal welding, ultrasonic welding, etc., to thereby form the diaper 1 in its underpants-shaped state as illustrated in FIG. 1. The side joining portion 2a is a joining portion disposed on one side in the left-right direction, whereas the side joining portion 2b is a joining portion disposed on the other side in the left-right direction.

The absorbent main body 10 includes: an absorbent body 11; a liquid-permeable top sheet 12 disposed further toward the skin side than the absorbent body 11; a second sheet 13 disposed between the top sheet 12 and the absorbent body 11; side sheets 14 disposed further toward the skin side than the absorbent body 11 and the second sheet 13; a liquid-impermeable back sheet 15 disposed further toward the non-skin side than the absorbent body 11; and an exterior sheet 16 disposed further toward the non-skin side than the back sheet 15.

The absorbent body 11 has a substantially rectangular planar-view shape, and includes: an absorbent core 11A for absorbing and retaining excreted fluid such as urine; and a liquid-permeable core-wrapping sheet (not illustrated) covering the absorbent core. Examples of the absorbent core 11A may include a molded product in which liquid-absorbent fibers such as pulp including superabsorbent polymer (SAP) are molded into a predetermined shape, such as a substantially hourglass-like planar-view shape (i.e., a molded product of liquid-absorbent fibers), and a so-called SAP sheet in which superabsorbent polymer (SAP) is shaped into a sheet form. Alternatively, the absorbent core 11A may be made by superposing, in the thickness direction, a SAP sheet and a molded product of liquid-absorbent fibers, and for example, the absorbent core 11A may have a configuration in which a SAP sheet, a molded product of liquid-absorbent fibers, and a SAP sheet are stacked in this order in the thickness direction from the skin side. The length, in the left-right direction, of the absorbent core 11A of the diaper 1 is substantially equal to the length of the absorbent body 11 in the left-right direction. Examples of the core-wrapping sheet may include liquid-permeable sheets such as tissue paper, nonwoven fabric, etc. It should be noted that the core-wrapping sheet does not have to be included.

As illustrated in FIG. 2, leg stretchable/contractible members 17 are disposed at both side portions, in the left-right direction, of the absorbent main body 10, to form leg gathers. The leg gathers of the diaper 1 in an underpants-shaped state are provided along each of the leg openings LH, and the stretching/contraction of the leg stretchable/contractible members 17 enables the diaper 1 to fit around the wearer's legs. A plurality of elastic strings are used as the leg stretchable/contractible members 17 of the diaper 1, and are fixed, for example, between the back sheet 15 and the exterior sheet 16 in a state stretched in the lengthwise direction of the absorbent main body 10 at a position between the two layers of the exterior sheet 16 formed by folding back the exterior sheet inwardly in the left-right direction.

Both side portions of the absorbent main body 10 in the left-right direction include leak-proof-wall stretchable/contractible members 18 disposed further inward than the leg stretchable/contractible members 17. The plurality of leak-proof-wall stretchable/contractible members 18 are fixed in a state stretched in the lengthwise direction between the two layers of each side sheet 14 formed by folding back each side sheet outwardly in the left-right direction, thereby forming a pair of leak-proof wall portions along the lengthwise direction. When the diaper 1 is worn, the stretchability/contractibility exerted by the leak-proof-wall stretchable/contractible members 18 causes the leak-proof wall portions to stand up toward the wearer's skin side and fit against the wearer's crotch section.

Examples of the leg stretchable/contractible members 17 and the leak-proof-wall stretchable/contractible members 18 may include elastic strings, stretchy sheets, etc.

The front waist portion 20 includes a skin-side sheet 21, a non-skin-side sheet 22, and a plurality of waist stretchable/contractible members 23 which are stretchable/contractible in the left-right direction. The skin-side sheet 21 and the non-skin-side sheet 22 are sheet members having substantially the same size and shape, and in the present embodiment, they have a substantially rectangular shape. The non-skin-side sheet 22 is disposed further toward the non-skin side than the skin-side sheet 21. For the skin-side sheet 21 and the non-skin-side sheet 22, it is possible to use, for example, SMS nonwoven fabric (spunbond/meltblown/spunbond nonwoven fabric), spunbond nonwoven fabric, air-through nonwoven fabric, point-bonded nonwoven fabric, stretchable nonwoven fabric, or a non-stretchable sheet such as a plastic sheet, a porous plastic sheet, or a laminated sheet thereof.

The plurality of waist stretchable/contractible members 23 of the front waist portion 20 are disposed side by side in the up-down direction between the skin-side sheet 21 and the non-skin-side sheet 22 and are fixed in a state stretched in the left-right direction. Thus, the front waist portion 20 can stretch/contract in the left-right direction and fit around the wearer's waist. Examples of the waist stretchable/contractible members 23 may include stretchable/contractible members such as rubber, spandex, etc. On the non-skin-side face of the non-skin-side sheet 22, the front waist portion 20 includes an engagement portion 25 with which an engaging portion 51 (described further below) of the fastening portion 5 is engageable. The engagement portion 25 is a so-called target region, which may be, for example, a nonwoven fabric sheet. It is preferable to select the engagement portion in accordance with the engaging portion 51; for example, in cases where a male member of a hook-and-loop fastener is used for the engaging portion 51, a female member of a hook-and-loop fastener may be used for the engagement portion 25. Furthermore, a separate member does not have to be provided on the non-skin-side face of the non-skin-side sheet 22 as the engagement portion 25, and instead, the non-skin-side sheet 22 may be composed of a member (e.g., nonwoven fabric) with which the engaging portion 51 is engageable, such that the engaging portion can directly engage with the non-skin-side face of the non-skin-side sheet 22. Note, however, that conversely, the engagement portion 25 of the front waist portion 20 may be a male member of a hook-and-loop fastener, and the engaging portion 51 of the fastening portion 5 (the surface opposing the front waist portion 20) may be a nonwoven fabric sheet or a female member of a hook-and-loop fastener.

The back waist portion 30 includes a skin-side sheet 31, a non-skin-side sheet 32, and a plurality of waist stretchable/contractible members 33 which are stretchable/contractible in the left-right direction. The skin-side sheet 31 and the non-skin-side sheet 32 are joined in a mutually stacked state using a joining means, such as an adhesive (e.g., hot-melt adhesive) or welding (e.g., thermal welding or ultrasonic welding). The skin-side sheet 31 and the non-skin-side sheet 32 are sheet members having substantially the same size and shape, and in the present embodiment, they have a substantially rectangular shape. The non-skin-side sheet 32 is disposed further toward the non-skin side than the skin-side sheet 31. For the skin-side sheet 31 and the non-skin-side sheet 32, it is possible to use, for example, SMS nonwoven fabric (spunbond/meltblown/spunbond nonwoven fabric) made of polypropylene, spunbond nonwoven fabric, air-through nonwoven fabric, point-bonded nonwoven fabric, stretchable nonwoven fabric, or a non-stretchable sheet such as a plastic sheet, a porous plastic sheet, or a laminated sheet thereof.

The plurality of waist stretchable/contractible members 33 of the back waist portion 30 are disposed side by side in the up-down direction between the skin-side sheet 31 and the non-skin-side sheet 32 and are fixed in a state stretched in the left-right direction. Thus, the front waist portion 20 and the back waist portion 30 can each stretch/contract in the left-right direction and fit around the wearer's waist. Examples of the waist stretchable/contractible members 33 may include stretchable/contractible members such as rubber, spandex, etc.

The fastening portion 5 is a so-called tab (grip) member and is a member to be used for forming a waist opening in cases where the front waist portion 20 has been cut. In the fastening portion 5 of the present embodiment, a base end portion (one side in the left-right direction in FIG. 2, etc.) of the fastening portion 5 is fixed at a position further toward the one side than the center of the front waist portion 20 in the left-right direction, and a leading end portion (the other side in the left-right direction in FIG. 2, etc.) of the fastening portion 5 includes an engaging portion 51 configured to detachably engage with the non-skin-side face of the front waist portion 20.

The fastening portion 5 is a member that is gripped by the wearer or a person attempting to put the diaper 1 on the wearer (also referred to hereinafter collectively as "wearer etc.") to perform certain actions. Therefore, it is preferable that the fastening portion 5 itself possesses sufficient strength to resist tearing. For the fastening portion 5, it is possible to use, for example, one of various films, e.g., a plastic film or a nylon film, nonwoven fabric, or a film obtained by laminating nonwoven fabric.

The engaging portion 51 is disposed on the skin-side face of the fastening portion 5, and is a member capable of detachably engaging with the non-skin-side face of the front waist portion 20 (more specifically, with the engagement portion 25). Examples of the engaging portion 51 may include a male member of a hook-and-loop fastener, an adhesive material such as an adhesive sheet, etc. The engaging portion 51 of the present embodiment is substantially rectangular, but it may have any shape, such as a round shape.

It should be noted that, as illustrated in FIG. 3, etc., the base end portion of the fastening portion 5 is fixed to the front waist portion 20 by a fixing portion F1 and an auxiliary fixing portion F2. The fixing portion F1 and the auxiliary fixing portion F2 of the present embodiment are welded portions in which the fastening portion 5 and the front waist portion 20 are joined together in the thickness direction by, for example, thermal welding or ultrasonic welding. The fastening portion 5 is fixed to the front waist portion 20 primarily by the fixing portion F1, and the auxiliary fixing portion F2 supports the fixing by the fixing portion F1.

### Cutting of Front Waist Portion 20:

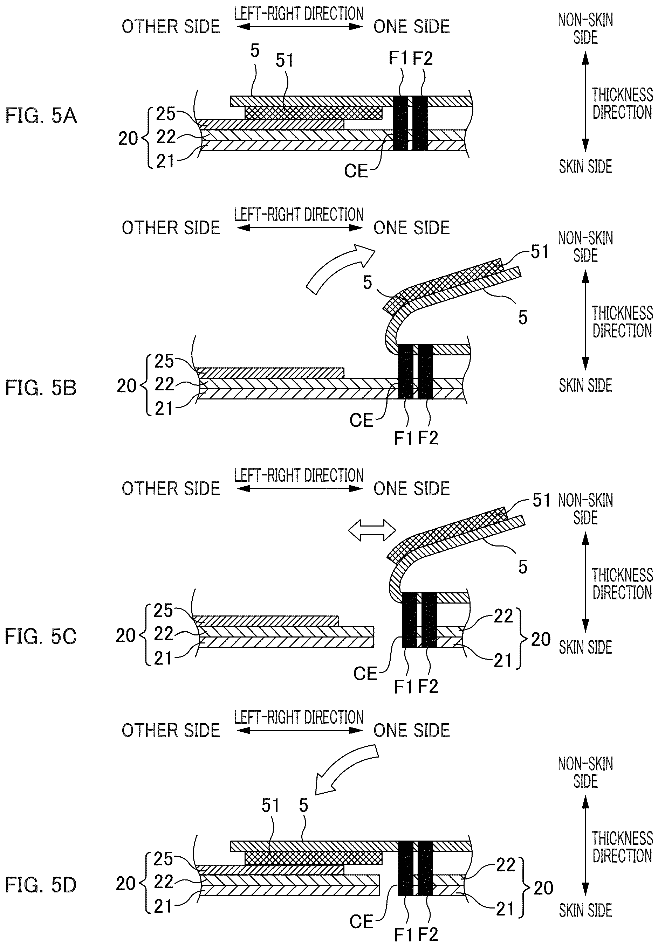

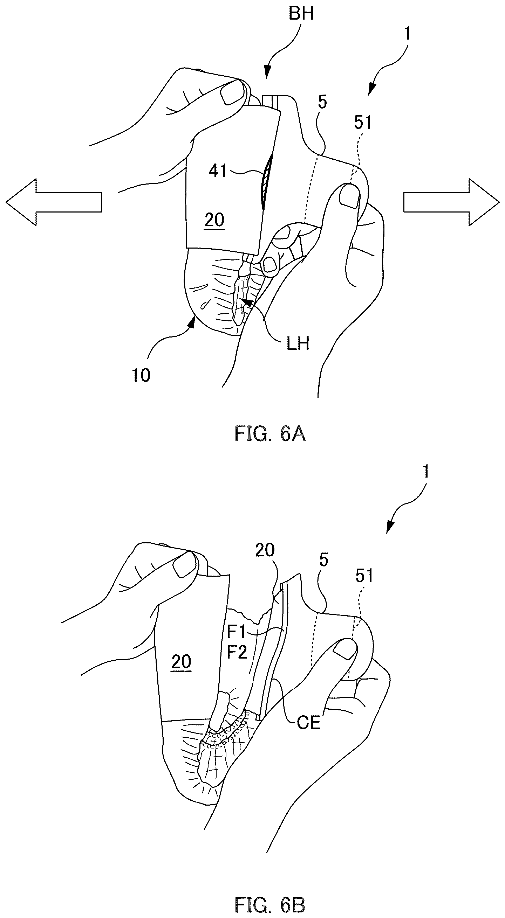

The cutting of the front waist portion 20 will be described with reference to FIGS. 5 and 6. FIG. 5 illustrates separation of the front waist portion 20 and formation of a waist opening. FIG. 6 illustrates actions for cutting the front waist portion 20. FIG. 5 illustrates configurations at a cross-section taken along line A-A of FIG. 3. It should be noted that FIGS. 5 and 6 are schematic diagrams, and the dimensions etc. therein are not necessarily accurate.

The diaper 1 is distributed in a state where the front waist portion 20 and the back waist portion 30 are joined by the side joining portions 2a, 2b. So, a user (e.g., parent/guardian) who has taken out the diaper 1 from a package will cut open a portion of the annular waist portion 20, 30 (in this example, a portion on one side, in the left-right direction, of the front waist portion 20) in the up-down direction. Then, the user passes one leg of a wearer (e.g., child) on one side (in this diaper 1, the wearer's right leg) through the leg opening LH on the other side in the left-right direction, and then pulls up the diaper 1 to the wearer's crotch section. Thereafter, the user engages the engaging portion 51 of the fastening portion 5 with the front waist portion 20 so as to fit around the wearer's waist, thereby forming a waist opening and thus achieving a worn state.

Thus, the diaper 1 can be worn in a state where one side in the left-right direction takes the form of a tape-type diaper (i.e., a state in which the leg opening LH is not formed), while the other side in the left-right direction takes the form of a so-called underpants-shaped diaper (i.e., a state in which the leg opening LH is formed).

Therefore, if one leg of the wearer can be successfully passed through the leg opening LH, a worn state can be achieved easily by engaging the fastening portion 5 (engaging portion 51) thereafter, even in the case of, for example, an infant/toddler who is kicking his/her legs, which would make it difficult to pass both legs through the leg openings LH, LH of an underpants-shaped disposable diaper. Further, in the case where the wearer himself/herself is putting on the diaper 1, it may be difficult to put on an ordinary tape-type diaper (i.e., a diaper having tapes on both sides in the left-right direction) in a bathroom etc.; in contrast, with a configuration where one side in the left-right direction has a tape-type form provided with the fastening portion 5 and the other side has an underpants-shaped form, the diaper can be put on easily, and the fit around the waist can also be adjusted.

Also after use of the diaper 1, the user can detach the fastening portion 5 from the front waist portion 20 and withdraw the leg from the leg opening LH on the other side in the left-right direction, or can separate the side joining portion 2b on the other side, thereby enabling the diaper 1 to be readily removed from the wearer's body.

To enable this, the diaper 1 includes a "cutting-guide portion 40" for guiding the cutting of the waist portion 20, 30 in the up-down direction. More specifically, the cutting-guide portion 40 is a portion that guides the user so that the waist portion 20, 30 can be cut in the up-down direction from the upper end (the end facing the waist opening BH) to the lower end (the end facing the leg opening LH). The cutting-guide portion 40 is also a portion where the waist portion 20, 30 can be cut readily. That is, the user can cut the waist portion 20, 30 at the cutting-guide portion 40 with a smaller force than when cutting the waist portion 20, 30 at a non-cutting-guide portion.

In the diaper 1 of the present embodiment, the fixing portion F1 where the front waist portion 20 and the fastening portion 5 are fixed functions as the cutting-guide portion 40. The fixing portion F1 is a welded portion in which the fastening portion 5 and the front waist portion 20 are joined together in the thickness direction by, for example, thermal welding or ultrasonic welding.

It should be noted that the auxiliary fixing portion F2 is provided further toward the side of the base end portion of the fastening portion 5 than the fixing portion F1 in the left-right direction. The auxiliary fixing portion F2 is also a welded portion in which the fastening portion 5 and the front waist portion 20 are joined together in the thickness direction by, for example, thermal welding or ultrasonic welding. The fastening portion 5 is fixed to the front waist portion 20 primarily by the fixing portion F1, and the auxiliary fixing portion F2 supports the fixing by the fixing portion F1. Note, however, that the diaper 1 does not have to include the auxiliary fixing portion F2.

FIG. 5A is a cross-sectional view of the diaper 1 in the underpants-shaped state illustrated in FIG. 1, etc. That is, in this state, the front waist portion 20 has not yet been cut, and the front waist portion 20 and the back waist portion 30 form a waist opening BH which is continuous via the side joining portions 2a, 2b. FIGS. 5B and 6A illustrate a state in which the leading end portion of the fastening portion 5 is gripped and pulled up toward the non-skin side from the state illustrated in FIG. 5A.

Then, as illustrated in FIG. 6A, by gripping the front waist portion 20 with one hand and gripping the fastening portion 5 with the other hand and pulling these portions respectively outward in the left-right direction, the front waist portion 20 is cut along the cutting-guide portion 40, as illustrated in FIGS. 5C and 6B. That is, the front waist portion 20 is separated into a one-side part including the fastening portion 5 and an other-side part not including the fastening portion 5. At the fixing portion F1, a portion of fibers of the skin-side sheet 21 and non-skin-side sheet 22 constituting the front waist portion 20 melts and integrates during welding. Therefore, the stiffness of the front waist portion 20 at the fixing portion F1 becomes higher than the stiffness at portions of the front waist portion 20 adjacent to the fixing portion F1. Thus, when the front waist portion 20 and the fastening portion 5 are gripped and pulled in the left-right direction by the user, the front waist portion 20 on the side having a lower grammage than the fastening portion 5 is cut so as to be torn at the boundary between portions with different stiffnesses. That is, the front waist portion 20 is cut along the other-side end CE, in the left-right direction, of the fixing portion F1.

Thereafter, as illustrated in FIG. 5D, the engaging portion 51 of the fastening portion 5 is engaged with the engagement portion 25 of the front waist portion 20, to form the waist opening of the diaper 1. The engaging portion 51 may be engaged with the engagement portion 25 such that the separated one-side part and other-side part, in the left-right direction, of the front waist portion 20 overlap each other in the front-back direction (thickness direction), or the engaging portion 51 may be engaged with the engagement portion 25 such that the separated parts do not overlap each other in the front-back direction. That is, the size of the waist opening of the diaper 1 can be adjusted by adjusting the position at which the engaging portion 51 is engaged.

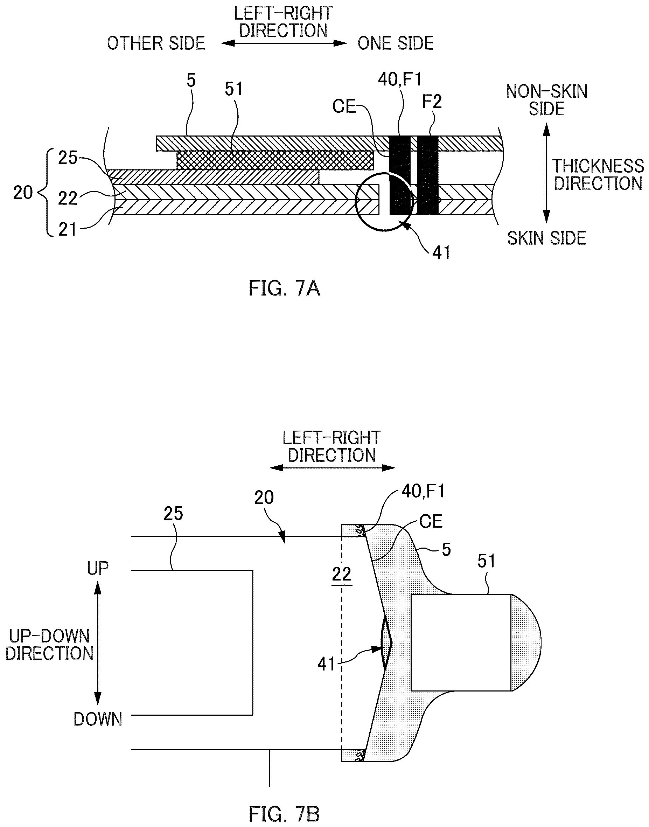

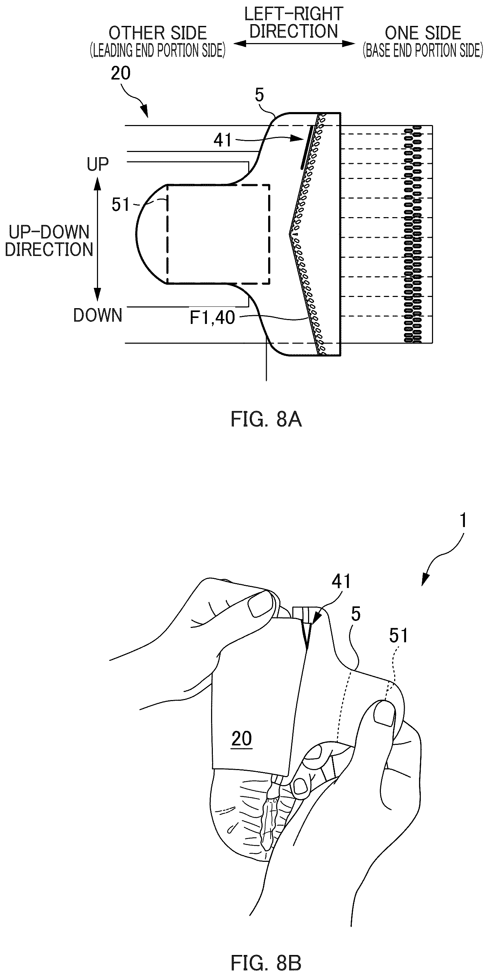

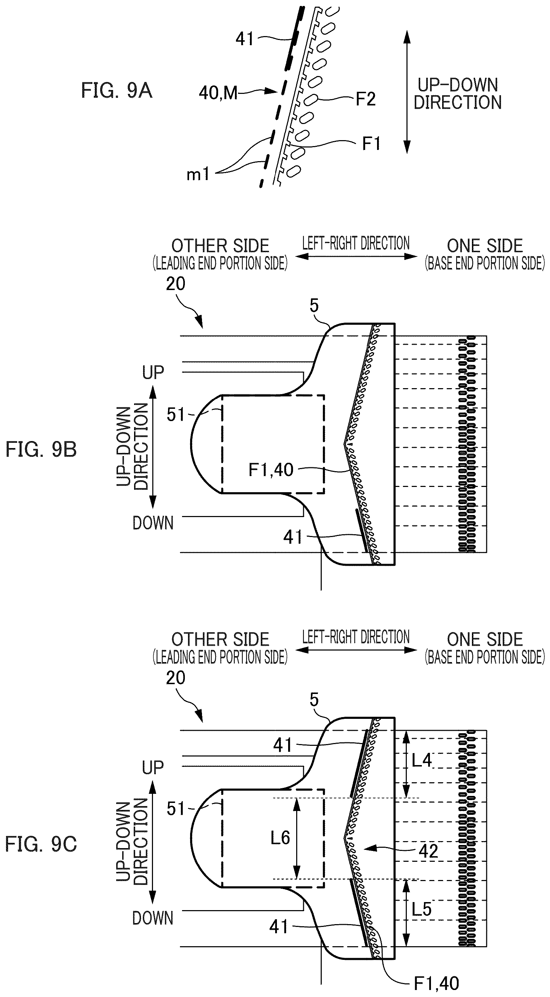

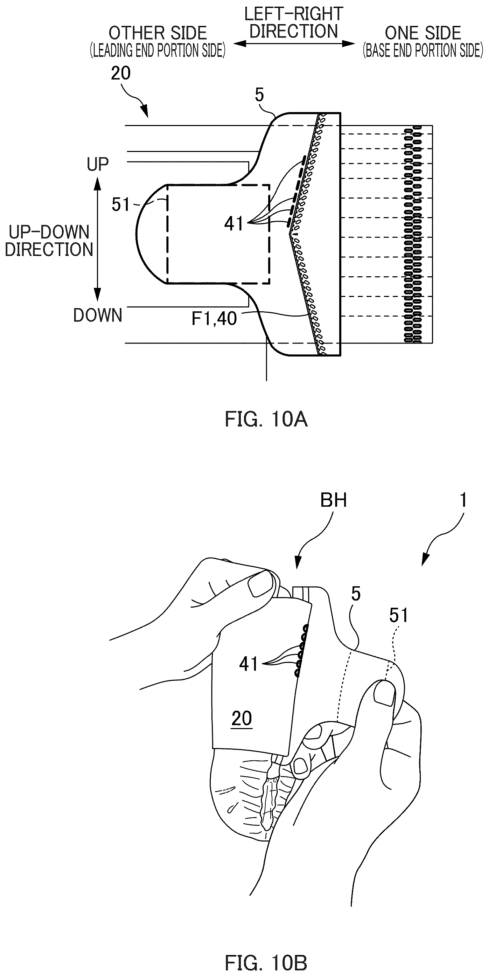

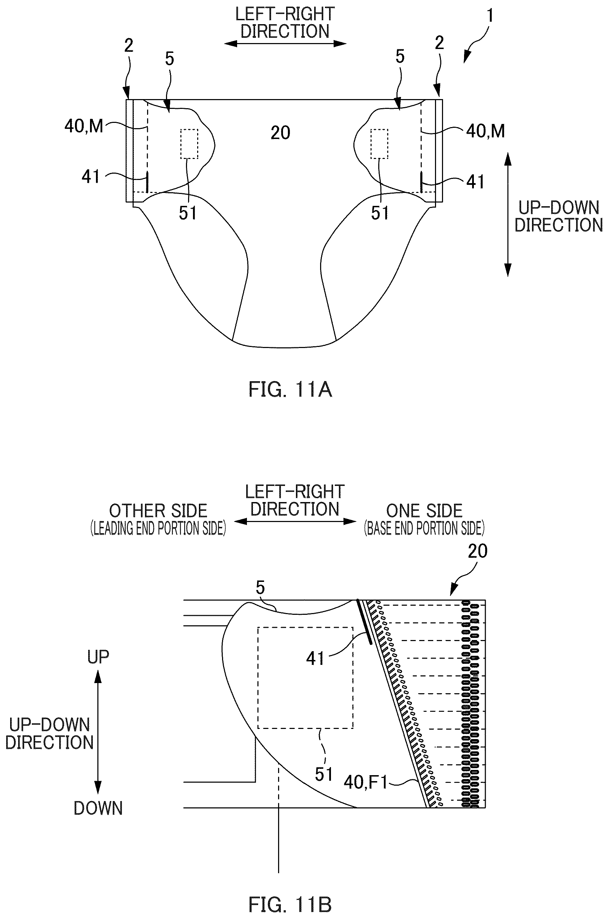

FIG. 7 illustrates a partially-cut portion 41. FIG. 7A is a diagram illustrating a cross-sectional configuration taken along line B-B in FIG. 3. FIG. 7B is a diagram illustrating a state in which the fastening portion 5 has been opened. FIG. 8 illustrates a modified example of the partially-cut portion 41. FIG. 9 illustrates modified examples of the cutting-guide portion 40 and the partially-cut portion 41. FIG. 10 illustrates a modified example of the partially-cut portion 41. FIG. 11 illustrates modified examples of the diaper 1.

The diaper 1 includes, in the cutting-guide portion 40, a "partially-cut portion 41" in which a portion of the waist portion 20, 30 (in this example, the front waist portion 20) in the up-down direction has been cut. Stated differently, a portion of the waist portion 20, 30 where a user performs cutting along the cutting-guide portion 40 is cut in advance. In the diaper 1 of the present embodiment, a portion of the front waist portion 20 adjacent to the fixing portion F1 on the other side in the left-right direction is cut in advance as the partially-cut portion 41. As illustrated in FIG. 7A, in the partially-cut portion 41, only the front waist portion 20 is cut, and the fastening portion 5 is not cut. It should be noted that FIG. 3 virtually illustrates the partially-cut portion 41 from the non-skin side of the fastening portion 5. The same applies to similar figures (FIG. 4, FIG. 8A, etc.) described further below.

For example, as illustrated in FIG. 3, in cases where the partially-cut portion 41 is provided in the central portion, in the up-down direction, of the cutting-guide portion 40, when a user lifts up the fastening portion 5, the partially-cut portion 41 is visually recognized as a through hole, as illustrated in FIGS. 6A and 7B. Further, as illustrated in FIG. 8A, in cases where the partially-cut portion 41 is provided at the upper end portion of the cutting-guide portion 40, when a user lifts up the fastening portion 5, the partially-cut portion 41 is visually recognized as a slit, as illustrated in FIG. 8B.

A user having visually recognized the partially-cut portion 41 can easily recognize that the waist portion 20, 30 is to be cut before attachment and also easily recognize the cutting position of the waist portion 20, 30. It is thus possible to prevent a situation where the waist portion 20, 30 is worn in an annular state without being cut. It is also possible to prevent a situation where the waist portion 20, 30 is cut not at the cutting-guide portion 40, but at a position separated from the fastening portion 5, for example, thereby preventing a reduction in the adjustment range of the waist portion 20, 30. That is, the partially-cut portion 41 allows the waist portion 20, 30 to be cut readily at the cutting-guide portion 40, making it easy to wear the diaper 1 properly. Thus, the diaper 1 including the fastening portion 5 can be employed advantageously.

Further, the diaper 1 including the annular waist portion 20, 30 can be manufactured according to the following method. First, while transporting a continuous material forming the front waist portion 20 and a continuous material forming the back waist portion 30 in a mutually superposed state in the thickness direction, side joining portions Sa, Sb are formed. Then, the continuous materials of the waist portion 20, 30 are cut into the individual product size. In this case, the constituent materials can be transported stably, thereby allowing mass production of the diapers 1, compared to cases where, for example, one of the left and right sides of the waist portion is left unjoined. The above manufacturing method, however, is merely an example, and the method for manufacturing the diaper 1 is not particularly limited.

Further, the cutting-guide portion 40 does not have to be the fixing portion F1. For example, a perforated line may be provided as the cutting-guide portion 40, separate from the fixing portion. In the perforated line M illustrated in FIG. 9A, joined portions and damaged portions m1 are lined up alternately. The damaged portions m1 may be small slits penetrating the waist portion 20, 30 in the thickness direction, or may be low-fiber portions in which the amount of fiber of the waist portion 20, 30 is small. Thus, the waist portion 20, 30 can be readily cut along the perforated line M. The length of the partially-cut portion 41 in the up-down direction is longer than the length of the damaged portion m1 in the up-down direction. That is, the partially-cut portion 41 is a portion formed by cutting one or more joined portions between the damaged portions m1 in the perforated line M. Another example of the cutting-guide portion 40 may be a portion in which a low-fiber portion-where the amount of fibers of the waist portion 20, 30 is small-extends continuously in the up-down direction.

Further, fixing between the waist portion 20, 30 and the fastening portion 5 at the fixing portion F1 and auxiliary fixing portion F2 does not have to be by welding, but may be fixing by an adhesive. Even in this case, the stiffness of the waist portion 20 at the fixing portion F1 is increased, so the waist portion 20, 30 can be readily cut along the fixing portion F1. Note, however, that fixing by a welded portion has a higher fixing strength, and thus the waist portion 20, 30 can be cut more readily along the fixing portion F1. It should be noted that the fixing method for the fixing portion F1 may be different from that for the auxiliary fixing portion F2.

The method for forming the partially-cut portion 41 is not particularly limited, and an example may include the following method. For example, in a step of manufacturing the diaper 1, when the constituent materials of the waist portion 20, 30 are transported with the left-right direction of the diaper 1 oriented in the transport direction, the tension can be adjusted so that a portion of the waist portion 20, 30 located in the cutting-guide portion 40 is cut, thereby forming the partially-cut portion 41. Alternatively, the partially-cut portion 41 may be formed by cutting a portion of the waist portion 20, 30 with a cutter after the step of forming the cutting-guide portion 40. Alternatively, the partially-cut portion 41 may be formed by varying the welding intensity (ultrasonic energy or welding temperature) depending on the position of the cutting-guide portion 40 in the up-down direction. Further, in cases where the cutting-guide portion 40 is a perforated line M, the partially-cut portion 41 may be formed by adjusting the pitch of the damaged portions m1 in the perforated line M.

Further, as in the diaper 1 of the modified example illustrated in FIG. 11A, the fastening portions 5 may be provided on both sides in the left-right direction, and not only on one side, and the diaper 1 may include two fastening portions 5. In the diaper 1 of FIG. 11A, the fastening portions 5 are fixed at the respective side joining portions 2 between the front waist portion and the back waist portion. Therefore, a perforated line M is provided as the cutting-guide portion 40, disposed further inward in the left-right direction than each side joining portion 2. Further, although not illustrated, the fastening portion 5 may be fixed to the back waist portion 30 by a fixing portion F1, or may engage with the back waist portion 30. Furthermore, the front waist portion 20 and the back waist portion 30 may be composed of a single continuous tubular member.

Further, the position in the up-down direction, the number and the length of the partially-cut portion 41 are not particularly limited. For example, in FIG. 3, the partially-cut portion 41 is provided at the central portion, in the up-down direction, of the cutting-guide portion 40, but in FIG. 8A, the partially-cut portion 41 is provided at the upper end portion of the front waist portion 20, whereas in FIG. 9B, the partially-cut portion 41 is provided at the lower end portion of the front waist portion 20. Alternatively, as illustrated in FIG. 9C, a plurality of partially-cut portions 41 may be provided by being spaced apart in the up-down direction.

As described above, in the diaper 1, the fixing portion F1 functions as the cutting-guide portion 40, and the cutting-guide portion 40 is the fixing portion F1. Alternatively, in cases where the cutting-guide portion 40 is to be provided separately from the fixing portion F1-for example, in cases where a welded portion or a perforated line separate from the fixing portion F1 is to be provided-it is preferable that at least a portion of the cutting-guide portion 40 is located, in the left-right direction, between the fixing portion F1 of the fastening portion 5 and an end 51e of the engaging portion 51 on the leading end portion side (the other side in the left-right direction) of the fastening portion 5.

With this configuration, on the other side of the cutting-guide portion 40 in the left-right direction, the fastening portion 5 is fixed to the front waist portion 20 by the engaging portion 51, whereas on the cutting-guide portion 40 or on the one side of the cutting-guide portion 40 in the left-right direction, the fastening portion 5 is fixed to the front waist portion 20 by the fixing portion F1. On a manufacturing line for the diaper 1 or during handling of the diaper 1 by a user, the waist portion 20, 30 is stretched in the left-right direction. Even in this case, with the aforementioned configuration, the fastening portion 5 can inhibit the stretching of the front waist portion 20 in the left-right direction. Thus, the partially-cut portion 41 can be inhibited from opening in the left-right direction, and unintended, unnecessary cutting of the waist portion 20, 30 starting from the partially-cut portion 41 can be prevented. For example, it is possible to prevent the entire range, in the up-down direction, of the waist portion 20, 30 from being cut along the cutting-guide portion 40 and prevent the waist portion 20, 30 from separating in the left and right directions. Thus, it is possible to prevent the production of defective items on the manufacturing line for the diaper 1 and the formation of wrinkles at the cut edge of the waist portion 20, 30 before the diaper 1 is used.

Note, however, that it will suffice if the cutting-guide portion 40 is provided further toward the leading end portion side of the fastening portion 5 than the fixing portion F1 of the fastening portion 5 in the left-right direction, and the configuration is not limited to the above. For example, the cutting-guide portion 40 may be located further toward the other side (inner side), in the left-right direction, than the fastening portion 5. However, by providing the cutting-guide portion 40 near the fixing portion F1, the adjustment range of the front waist portion 20 can be increased.

Further, as illustrated in FIG. 3, it is preferable that, in the stretched state of the waist portion 20, 30, at least a portion of the partially-cut portion 41 overlaps the engaging portion 51 of the fastening portion 5 in the up-down direction.

With this configuration, the partially-cut portion 41 is located on an extension, in the left-right direction, of the portion where the fastening portion 5 is fixed to the front waist portion 20 by the engaging portion 51. That is, the partially-cut portion 41 is located in a region where the stretching of the front waist portion 20 in the left-right direction is more likely to be inhibited by the fastening portion 5. Thus, the partially-cut portion 41 can be inhibited from opening in the left-right direction, thereby preventing unnecessary cutting of the waist portion 20, 30 at the cutting-guide portion 40.

Further, when a user lifts up the fastening portion 5, force is likely to concentrate on the engaging portion 51. Therefore, the user's action of lifting up the fastening portion 5 is likely to apply force also to the partially-cut portion 41, which is located on an extension of the engaging portion 51. Thus, the front waist portion 20 can be cut readily in one movement along the cutting-guide portion 40, starting from the partially-cut portion 41. This makes it easy for the user to readily cut the front waist portion 20.

Note, however, that contrary to the above, as illustrated in the modified example of FIG. 8A, in the stretched state of the waist portion 20, 30, the partially-cut portion 41 does not have to overlap the engaging portion 51 of the fastening portion 5 in the up-down direction.

In this case, the partially-cut portion 41 will be provided in a region of the front waist portion 20 where the restriction of movement by the engaging portion 51 is small. Therefore, the position of the front waist portion 20 is likely to shift relative to the fastening portion 5, making the partially-cut portion 41 more readily visible from beneath the fastening portion 5. Thus, a user can visually recognize the partially-cut portion 41 more readily before lifting up the fastening portion 5. Thus, the user can promptly and readily recognize that the front waist portion 20 is to be cut before attachment and also easily recognize the cutting position of the front waist portion 20.

It should be noted that the waist portion 20, 30 includes waist stretchable/contractible members 23, 33 which stretch/contract in the left-right direction. Therefore, the positional relationship among the various portions is verified in the stretched state in which the annular waist portion 20, 30 has been stretched so that wrinkles are eliminated, and not in the natural state of the diaper 1.

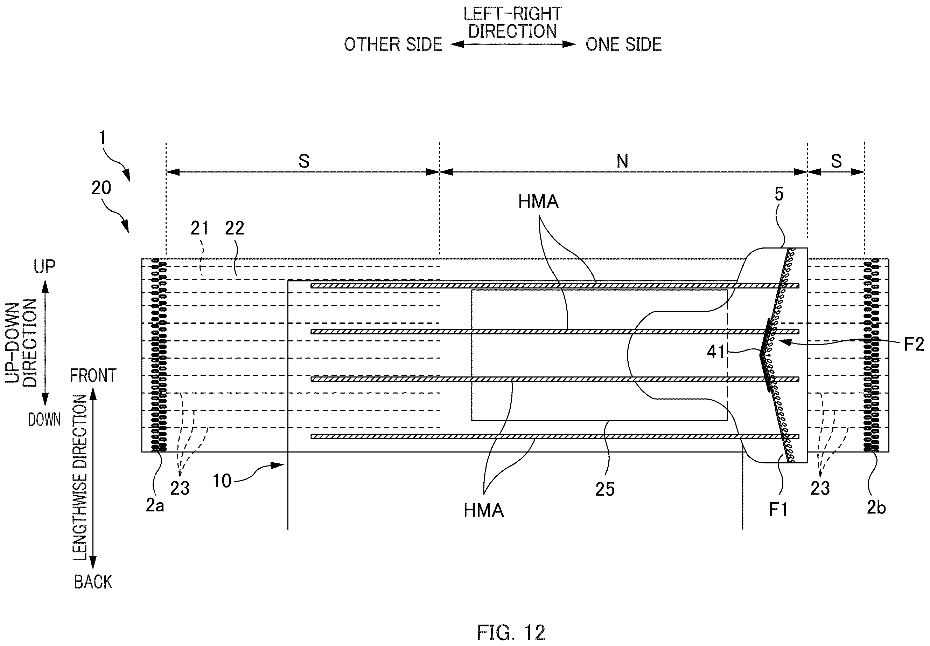

Further, the diaper 1 of the present embodiment includes: a non-stretchable/contractible region N (a region that does not exhibit stretchability/contractibility) in which the waist stretchable/contractible members 23 are not present from the upper end to the lower end of the front waist portion 20; and a stretchable/contractible region S in which the waist stretchable/contractible members 23 are present and in which the front waist portion 20 stretches/contracts in the left-right direction. As illustrated in FIG. 12 described below, the non-stretchable/contractible region N primarily overlaps with the position where the engagement portion 25 is provided. Thus, when a user grips the fastening portion 5 with one hand and the engagement portion 25 or its vicinity with the other hand, the engagement portion 25 is less likely to stretch in the left-right direction, thereby making it easier to cut the front waist portion 20 at the cutting-guide portion 40. Also thereafter, the user can stably bring the fastening portion 5 close to the engagement portion 25 for engagement. Note, however, that the diaper 1 does not have to include the non-stretchable/contractible region N.

Further, as illustrated in FIG. 3, it is preferable that at least a portion of the partially-cut portion 41 overlaps the fastening portion 5 in the front-back direction (thickness direction).

With this configuration, the partially-cut portion 41 is covered by the fastening portion 5, thereby inhibiting external force from being applied to the partially-cut portion 41, and also inhibiting unnecessary cutting of the front waist portion 20 starting from the partially-cut portion 41. Further, the partially-cut portion 41 is visually recognized at the timing when the user lifts up the fastening portion 5 to cut the front waist portion 20, i.e., at the timing when the diaper 1 is to be attached. Thus, the user can readily recognize that the front waist portion 20 is to be cut at the partially-cut portion 41 (cutting-guide portion 40).

Further, it is preferable that the waist portion 20, 30 includes, in the cutting-guide portion 40, a non-cut portion 42 in which the waist portion 20, 30 has not been cut, and that, in the stretched state of the waist portion 20, 30, the length of the partially-cut portion 41 in the up-down direction is different from the length of the non-cut portion 42 in the up-down direction.

It should be noted that, although the fastening portion 5 in the present embodiment is longer than the front waist portion 20 in the up-down direction, the length of the partially-cut portion 41 and the length of the non-cut portion 42 refer to their length within a range where the waist portion 20, 30 and the fastening portion 5 overlap each other. Further, in cases where a plurality of partially-cut portions 41 are provided with intervals therebetween in the up-down direction, the total length of the plurality of partially-cut portions 41 is compared. Similarly, in cases where a plurality of non-cut portions 42 are provided with intervals therebetween in the up-down direction, the total length of the plurality of non-cut portions 42 is compared.

For example, in the cutting-guide portion 40 of FIG. 3, the length L1 of the partially-cut portion 41 in the up-down direction is shorter than the total length (L2+L3) of the two non-cut portions 42 in the up-down direction (L1 < L2+L3). In this case, unnecessary cutting of the front waist portion 20 starting from the partially-cut portion 41 can be inhibited.

Conversely, in the cutting-guide portion 40 of FIG. 9C, the length (L4+L5) of the plurality of partially-cut portions 41 in the up-down direction is longer than the length L6 of the non-cut portion 42 (L4+L5 > L6). In this case, the partially-cut portion 41 and cutting-guide portion 40 can be readily recognized by a user. Thus, the user can easily cut the front waist portion 20 along the cutting-guide portion 40. Further, the length of the front waist portion 20 that requires cutting by the user can be reduced, thereby facilitating the cutting operation.

Further, in cases where the fastening portion 5 extends outward in the up-down direction from the front waist portion 20, it is preferable that the cutting-guide portion 40 (fixing portion F1) is formed also in the extension portion. With this configuration, a user can be more aware to cut the front waist portion 20 over a long length in the up-down direction and can cut the front waist portion 20 more securely.

FIG. 12 is a diagram illustrating an adhesive HMA joining the skin-side sheet 21 and non-skin-side sheet 22. The waist portion 20, 30 is formed by a plurality of sheet members (the skin-side sheets 21, 31 and non-skin-side sheets 22, 32). Each of the sheet members is joined by an adhesive (e.g., hot-melt adhesive) with the sheet member adjacent thereto in the thickness direction of the waist portion 20, 30. In the diaper 1, as illustrated in FIG. 12, an adhesive HMA is applied between the skin-side sheet 21, 31 and the non-skin-side sheet 22, 32 to join these sheets together.

It is preferable that the adhesive HMA reaches at least an end of the partially-cut portion 41 located on the one side in the left-right direction. The end, in the left-right direction, of the partially-cut portion 41 has a certain length in the up-down direction, so it will suffice if the adhesive HMA reaches at least a portion of the end of the partially-cut portion 41 which extends in the up-down direction. In FIG. 12, the adhesive HMA is applied in four lines extending linearly in the left-right direction with intervals therebetween in the up-down direction. Further, the adhesive HMA is applied so as to extend across the partially-cut portion 41 in the left-right direction. Thus, the adhesive HMA reaches both the one-side end and the other-side end, in the left-right direction, of the front waist portion 20 which has been cut at the partially-cut portion 41. Thus, it is possible to inhibit the sheet members of the front waist portion 20 from peeling and opening up at the partially-cut portion 41. That is, it is possible to inhibit deterioration in visual recognizability of the partially-cut portion 41 (through hole or slit) due to the skin-side sheet 21 and non-skin-side sheet 22 shifting relative to each other at the partially-cut portion 41.

Further, it is preferable that the adhesive HMA is located so as to extend across the cutting-guide portion 40 in the left-right direction. It will suffice if the adhesive HMA extends across at least a portion of the cutting-guide portion 40, which extends along the up-down direction. The stiffness is increased at the intersection portion between the cutting-guide portion 40 and the adhesive HMA. Therefore, even if the front waist portion 20 is unintentionally cut along the cutting-guide portion 40 starting from the partially-cut portion 41, unnecessary cutting at the intersection portion between the cutting-guide portion 40 and the adhesive HMA can be inhibited. Thus, it is possible to inhibit the entire width of the front waist portion 20 in the up-down direction from being cut and inhibit the front waist portion 20 from being separated. It should be noted that the number or length of the region in which the adhesive HMA is applied, as illustrated in FIG. 12, is merely an example and is not limited thereto.

The cutting-guide portion 40 (fixing portion F1) illustrated in FIG. 3 includes an upper inclined portion 40A that is inclined upwardly from the central portion in the up-down direction and toward the base end portion side (the one side in the left-right direction) of the fastening portion 5, and a lower inclined portion 40B that is inclined downwardly from the central portion in the up-down direction and toward the base end portion side of the fastening portion 5. In this case, when a user grips the front waist portion 20 and the fastening portion 5 and pulls them in the left-right direction, tensile force concentrates on a cutting start point K. The cutting start point K is the leading end portion (the other-side end in the left-right direction) of the horizontal V-shaped cutting-guide portion 40. That is, the cutting start point is the intersection point between the upper inclined portion 40A and the lower inclined portion 50B. Force is transmitted from the cutting start point K along the upper inclined portion 40A and the lower inclined portion 40B, thereby cutting the front waist portion 20.

In the above case, it is preferable that the partially-cut portion 41 is located so as to extend across the cutting start point K, i.e., the intersection point between the upper inclined portion 40A and the lower inclined portion 40B of the cutting-guide portion 40. That is, it is preferable that the partially-cut portion 41, where the front waist portion 20 has already been cut, is located in a region where the user's tensile force is concentrated. With this configuration, the user can easily cut the front waist portion 20 along the cutting-guide portion 40 even with a small force.

It should be noted that the shape of the cutting-guide portion 40 is not limited to that illustrated in FIG. 3. For example, as illustrated in FIG. 11B, the cutting-guide portion 40 (fixing portion F1) may be inclined at a predetermined angle, or may be a cutting-guide portion 40 along the up-down direction, although not illustrated. In this case, the upper end portion or the lower end portion of the front waist portion 20 becomes the cutting start point K. Thus, it is preferable that the partially-cut portion 41 is provided at least at either the upper end portion or the lower end portion of the front waist portion 20.

As illustrated in FIG. 4, the fastening portion 5 includes a grip portion 5t. The grip portion 5t is a portion of the fastening portion 5 disposed further toward the leading end portion side (the other side in the left-right direction) than the engaging portion 51. A user can easily grip the portion of the fastening portion 5 that is not joined to the front waist portion 20 by the engaging portion 51.

Further, in the stretched state of the waist portion 20, 30, it is preferable that at least a portion of the partially-cut portion 41 overlaps the grip portion 5t in the up-down direction. With this configuration, the partially-cut portion 41 is located on an extension of the the grip portion 5t in the left-right direction. Therefore, when a user cuts the cutting-guide portion 40 while gripping the grip portion 5t and lifting up the fastening portion 5, the tensile force from the grip portion 5t can be readily applied to the partially-cut portion 41. Thus, the front waist portion 20 can be readily cut along the cutting-guide portion 40, starting from the partially-cut portion 41.

Further, as illustrated in FIG. 7B, it is preferable that the color of the skin-side face (inner face) of the fastening portion 5 is different from the color of the non-skin-side face (outer face) of the waist portion 20, 30. With this configuration, when a user lifts up the fastening portion 5, the color of the inner face of the fastening portion 5 (e.g., green, blue, etc.) becomes visually recognizable from a portion where the partially-cut portion 41 has been opened in the left-right direction. On the skin-side face (which is white, for example) of the front waist portion 20, the different-color portion will stand out. This can make a user focus on the partially-cut portion 41 more readily and recognize the partially-cut portion 41 more readily. Thus, the user can recognize that the waist portion 20, 30 is to be cut before attachment and also recognize the cutting position of the waist portion 20, 30.

It should be noted that it will suffice if the color difference is of a degree where the color of the skin-side face of the adjacent fastening portion 5 and the color of the non-skin-side face of the front waist portion 20 can be distinguished by visual inspection. More specifically, the color difference between the skin-side face of the fastening portion 5 and the non-skin-side face of the front waist portion 20 may be, for example, such that the color difference (ΔE76), which is calculated according to the color difference formula within the L*a*b* color space in conformity with CIE 1976, is 3.2 or greater, preferably 6.5 or greater, more preferably 13.0, even more preferably 25.0 or greater, in accordance with grades based on color tolerance generally adopted in the Japanese Industrial Standards (JIS) or by various other industry associations. Color difference measurement can be conducted with a general-purpose colorimeter (for example, CR-400 from Konica Minolta, Inc., or another equivalent device) in a thermoregulated chamber set to 20 degrees.

Further, the waist portion 20, 30 is formed by one or more sheet members. It is preferable that the tensile strength of the one or more sheet members in the left-right direction is higher than the tensile strength of the same one or more sheet members in the up-down direction. In the diaper 1 of the present embodiment, the cutting-guide portion 40 is provided in the front waist portion 20, and the front waist portion 20 is formed by two sheet members, i.e., the skin-side sheet 21 and the non-skin-side sheet 22. It should be noted that, although the front waist portion 20 also includes the waist stretchable/contractible members 23, the tensile strength is compared in a state where the waist stretchable/contractible members 23 have been removed.

When the tensile strength, in the left-right direction, of the sheet members (skin-side sheet 21 and non-skin-side sheet 22) constituting the front waist portion 20 is higher than the tensile strength in the up-down direction, the front waist portion 20 is less likely to stretch in the left-right direction, as compared with the reverse case. Thus, the partially-cut portion 41 can be inhibited from opening in the left-right direction, thereby inhibiting unnecessary cutting of the front waist portion 20 starting from the partially-cut portion 41.

The tensile strength of the sheet members (21, 22) can be compared by a known method using a tensile tester (e.g., Model 5965 from Instron, or another equivalent device). For example, the constituent materials of the diaper 1 are separated using a cold spray, etc., and then the one or more sheet members to be measured (in this example, the skin-side sheet 21 and the non-skin-side sheet 22) are taken out. The target sheet members are cut into a predetermined size (e.g., a rectangular shape whose lengthwise direction is oriented in the measurement direction; 160 mm long and 25 mm wide), to prepare respective samples. That is, a sample whose lengthwise direction is in the up-down direction of the diaper 1 and a sample whose lengthwise direction is in the left-right direction of the diaper 1 are prepared. In cases where the waist portion 20, 30 is formed by a plurality of sheet members, measurement is conducted with the plurality of sheet members in a layered state. For example, the sample of the present embodiment is a material in which the skin-side sheet 21 and the non-skin-side sheet 22 are layered, with the waist stretchable/contractible members 23 removed. Both ends of the sample are pinched with respective chucks of the tensile tester (chuck-to-chuck distance: 100 mm), and the strength (tensile load; N/25 mm) for stretching the sample at a tensile speed of 100 mm/minute up to an arbitrary length, such as 105% of the sample's maximum stretched state, is measured. Then, the fact that the strength when the sample is stretched in the left-right direction is higher (i.e., the tensile load is greater) than the strength when the sample is stretched in the up-down direction is verified.

Further, it is preferable that the skin-side sheet 21 and the non-skin-side sheet 22 forming the front waist portion 20 are spunbond nonwoven fabric, and include embossed-joining portion(s) formed by embossing the fibers constituting the sheets. With this configuration, the thickness at the edges of the skin-side sheet 21 and non-skin-side sheet 22 at the partially-cut portion 41 can be suppressed, thereby improving the visual recognizability of the partially-cut portion 41.

Further, since the fibers are molten and integrated at the embossed-joining portions, the sheets become harder to cut. Thus, by providing such embossed-joining portions in a portion of the skin-side sheet 21 and non-skin-side sheet 22, unnecessary cutting of the front waist portion 20 starting from the cutting-guide portion 40 can be inhibited.

In contrast, at non-joining portions where no emboss joining is conducted, the sheets can be cut easily. Therefore, when the skin-side sheet 21 and the non-skin-side sheet 22 include non-joining portions, rather than including the embossed-joining portions over the entire surface of the skin-side sheet 21 and the non-skin-side sheet 22, the partially-cut portion 41 becomes easier to form. Further, a user can cut the front waist portion 20 along the cutting-guide portion 40 more easily.

Further, when the front waist portion 20 is viewed in the thickness direction, it is preferable that at least a portion of the embossed-joining portions of the skin-side sheet 21 is misaligned from the embossed-joining portions of the non-skin-side sheet 22. With this configuration, it is possible to prevent the embossed-joining portions of the skin-side sheet 21 and the embossed-joining portions of the non-skin-side sheet 22 from completely overlapping one another and thereby forming regions where the sheets are extremely hard to cut. It is also possible to prevent the non-joining portions of the skin-side sheet 21 and the non-joining portions of the non-skin-side sheet 22 from completely overlapping one another and thereby forming regions where the sheets are extremely easy to cut.

Further, the waist portion 20, 30 is formed by one or more sheet members, and also the fastening portion 5 is formed by one or more fastening-portion sheet members. It is preferable that the maximum value of the total grammage of the one or more sheet members is smaller than the minimum value of the total grammage of the one or more fastening-portion sheet members. "Grammage" refers to the weight per unit area (g/m²).

For example, in the diaper 1 of the present embodiment, the cutting-guide portion 40 is provided in the front waist portion 20, and the front waist portion 20 is formed by two sheet members, i.e., the skin-side sheet 21 and the non-skin-side sheet 22. Further, although not illustrated, in the waist portion, the non-skin-side sheet 22 is folded back to form a three-layer structure, whereas in the other regions, a two-layer structure is formed. In this case, the maximum value of the total grammage refers to the total value of the grammage of the skin-side sheet 21 and twice the grammage of the non-skin-side sheet 22. On the other hand, although not illustrated, the fastening portion 5 is formed by three layers of fastening-portion sheet members, in which both faces of a plastic film are respectively sandwiched by nonwoven fabrics having the same size, and the entire fastening portion 5 has the same structure. In this case, the minimum value of the total grammage refers to the total value of the grammage of the skin-side nonwoven fabric, the grammage of the plastic film, and the grammage of the non-skin-side nonwoven fabric.

The aforementioned configuration-wherein the total grammage (maximum value) of the sheet members (skin-side sheet 21 and non-skin-side sheet 22) constituting the front waist portion 20 is smaller than the total grammage (minimum value) of the fastening-portion sheet members-means that the amount of resin (the fibers in the nonwoven fabrics, the resin in the film, etc.) in the sheet members constituting the front waist portion 20 is smaller than that of the fastening portion 5. Thus, as illustrated in FIG. 6, when a user grips the front waist portion 20 and the fastening portion 5 and pulls them in the left-right direction, the fastening portion 5 having a larger total grammage (larger amount of resin) can maintain its fixed state by the fixing portion F1, whereas the front waist portion 20 having a smaller total grammage (smaller amount of resin) can be readily cut so as to be torn at the cutting-guide portion 40. Thus, the user can easily cut and separate the front waist portion 20. It is also easy to form the partially-cut portion 41.

Comparison of the total grammage of the sheet members and that of the fastening-portion sheet members can be measured by a known method. First, the fastening portion 5 is removed from the diaper 1, and then the engaging portion 51 is removed from the fastening portion 5 with a cold spray, etc. In cases where the structure of the materials is the same over the entire fastening portion 5 (e.g., in a three-layer structure), the fastening portion 5 is cut out in a predetermined size (e.g., a 10×10 mm square) to prepare a sample, and from the sample's weight and area, the total grammage is calculated as the minimum value of the total grammage. In cases where the fastening portion 5 has different structures in different regions, the total grammage is calculated for each region, to find the minimum value.

Similarly, the total grammage of the sheet members forming the waist portion 20, 30 can be found by cutting out, as a sample, a region of the waist portion (in this example, the front waist portion 20) in which the cutting-guide portion 40 is formed, and a region of the front waist portion 20 having the same material configuration. For example, a region from the upper end to the lower end of the front waist portion 20 and having a width of 10 mm in the left-right direction is cut out as a sample. If the cut-out region includes waist stretchable/contractible members 23, the waist stretchable/contractible members 23 are removed from the cut-out region, to prepare a sample. In cases where the structure of the materials is the same over the entire sample (e.g., in a two-layer structure composed of the skin-side sheet 21 and the non-skin-side sheet 22), the total grammage is calculated from the sample's weight and area, as the maximum value of the total grammage. In cases where the sample has different structures in different regions (for example, in cases where the waist portion includes a folded-back region having a three-layer structure, and a two-layer structure in other regions), the total grammage is calculated for each region, to find the maximum value.

Further, as illustrated in FIG. 4, it is preferable that, on the side (the one side) further toward the base end portion of the fastening portion 5 than the cutting-guide portion 40 in the left-right direction, the diaper 1 includes joining portions 60 that join the fastening portion 5 and the waist portion 20, 30. Further, it is preferable that, in the stretched state of the waist portion 20, 30, at least a portion of the partially-cut portion 41 overlaps the joining portions 60 in the up-down direction.

With this configuration, fixing of the fastening portion 5 to the front waist portion 20 is reinforced by the joining portions 60. Thus, the fastening portion 5 can inhibit stretching of the front waist portion 20 in the left-right direction. Particularly, when the joining portions 60 are located on an extension of the partially-cut portion 41 in the left-right direction, stretching of the front waist portion 20 at the partially-cut portion 41 can be inhibited, thereby inhibiting the partially-cut portion 41 from opening toward the left and right. Thus, unnecessary cutting of the front waist portion 20 along the cutting-guide portion 40 can be inhibited.

In the present embodiment, the joining portions 60 may be, for example, welded portions formed by thermal welding. Note, however, that the joining method for the joining portions 60 is not particularly limited, and for example, it is possible to employ, for example, joining by ultrasonic welding, joining by non-heating compression, joining by an adhesive, etc. Further, by providing the joining portions 60, the fastening portion 5, other than the fixing portion F1 and the engaging portion 51, can be prevented from lifting up. Thus, it is possible to inhibit detachment or folding of the fastening portion 5 on a manufacturing line for the diapers 1. Therefore, as illustrated in FIG. 4, the joining portions 60 may also be provided in a region located further toward the leading end portion side, in the left-right direction, than the cutting-guide portion 40 and where the engaging portion 51 is not present. It should be noted that the fixing by the joining portions 60 is weaker than the fixing by the fixing portion F1, and therefore, a user can lift up and peel the fastening portion 5.

Further, as illustrated in the modified example of FIGS. 10A and 10B, the partially-cut portion 41 may be located further inward than an end of the waist portion 20, 30 in the up-down direction. With this configuration, the partially-cut portion 41 will be visually recognized as a through hole in the waist portion 20, 30, and not as a slit in the upper or lower end of the waist portion 20, 30. Thus, the partially-cut portion 41 can be easily recognized as an intentionally cut portion serving as the cutting position of the waist portion 20, 30.

Further, the diaper 1 may include a plurality of partially-cut portions 41 lined up in the up-down direction. That is, a plurality of partially-cut portions 41 may be lined up intermittently in the up-down direction. With this configuration, when a user lifts up the fastening portion 5, the plurality of partially-cut portions 41 open up in the left-right direction and are visually recognized as a perforated line composed of a plurality of through holes. Thus, the partially-cut portion 41 can be more readily recognized as the cutting position of the waist portion 20, 30.

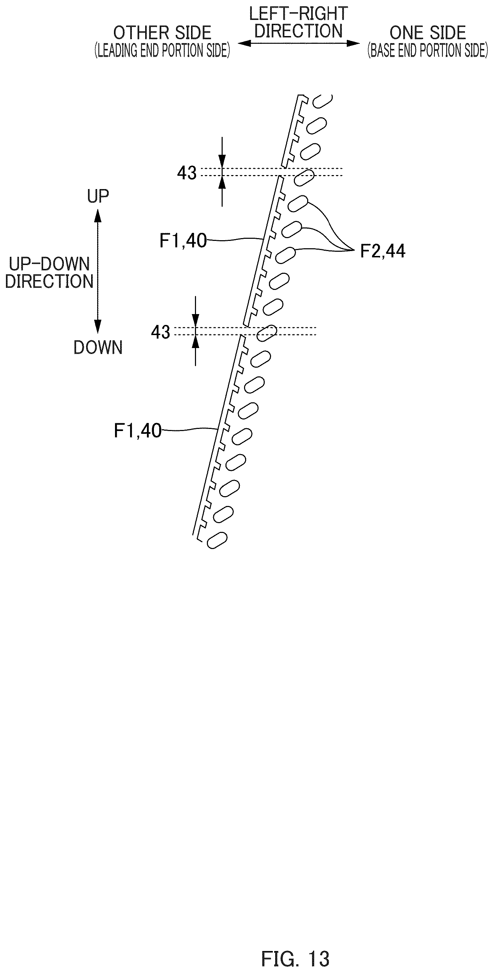

FIG. 13 is a diagram illustrating a modified example of the cutting-guide portion 40. The diaper 1 may include non-cutting-guide portion(s) 43 at least on one side, in the up-down direction, of the cutting-guide portion 40. In the present embodiment, the fixing portion F1 formed by welding serves as the cutting-guide portion 40. So, non-welded portions are provided as non-cutting-guide portions 43. At the non-welded portion, the waist portion 20, 30 is less likely to be cut, compared to the welded portion. In cases where the cutting-guide portion 40 is a perforated line, a portion where the perforated line is not formed serves as the non-cutting-guide portion 43.

According to the above, unnecessary cutting of the front waist portion 20 starting from the partially-cut portion 41 can be readily stopped by the non-cutting-guide portion 43. Further, also when forming the partially-cut portion 41 on a manufacturing line for manufacturing the diapers 1, the formation position, length, etc., of the partially-cut portion 41 can be readily adjusted by adjusting the arrangement of the non-cutting-guide portion 43. Thus, it becomes easier to form a desired partially-cut portion 41.

It will suffice if the non-cutting-guide portion 43 is provided at least on one side, in the up-down direction, of the cutting-guide portion 40. Therefore, the non-cutting-guide portion 43 may be provided between cutting-guide portions 40 lined up in the up-down direction as illustrated in FIG. 13, or may be provided on one side of the cutting-guide portion 40 in the up-down direction, i.e., at the upper end portion or lower end portion of the waist portion 20, although not illustrated. There may be one or more non-cutting-guide portions 43.

Note, however, that the welded portion may be continuous from the upper end to the lower end of the front waist portion 20, like the cutting-guide portion 40 illustrated in FIG. 3. In this case, a user can smoothly cut the waist portion 20, 30 along the cutting-guide portion 40.

Furthermore, in cases where the non-cutting-guide portion 43 is provided, it is preferable to provide a welded portion 44 adjacent to the cutting-guide portion 40 on the base end portion side of the fastening portion 5 in the left-right direction (i.e., on the one side in the left-right direction). The welded portion 44 is a portion formed by welding the waist portion 20, 30 and the fastening portion 5. In the present embodiment, the auxiliary fixing portion F2 is provided, as the welded portion 44, adjacent to the cutting-guide portion 40 (fixing portion F1). In the stretched state of the waist portion 20, 30, it is preferable that at least a portion of the non-cutting-guide portion 43 overlaps the welded portion 44 (auxiliary fixing portion F2) in the up-down direction.

With this configuration, at the welded portion 44, due to the difference in stiffness from its adjacent region, the waist portion 20, 30 can be readily cut (i.e., the fibers can be readily torn) as described above. Therefore, although the waist portion 20, 30 resists cutting at the non-cutting-guide portion 43, the adjacent welded portion 44 (auxiliary fixing portion F2) can assist cutting of the waist portion 20, 30. Thus, a user can smoothly cut the waist portion 20, 30 by the cutting-guide portion 40 and the welded portion 44. It should be noted that, also in cases where the cutting-guide portion 40 is a perforated line M, the welded portion 44 can assist cutting of the waist portion 20, 30.

Further, in the present embodiment, the welded portions 44 (auxiliary fixing portions F2) are lined up intermittently with intervals therebetween in the up-down direction. Therefore, when a user cuts and separates the front waist portion 20, the side further toward the base end portion of the fastening portion 5 (the one side in the left-right direction) than the cutting-guide portion 40 can be prevented from being cut by mistake along the auxiliary fixing portions F2.

### Package 70 of Diapers 1:

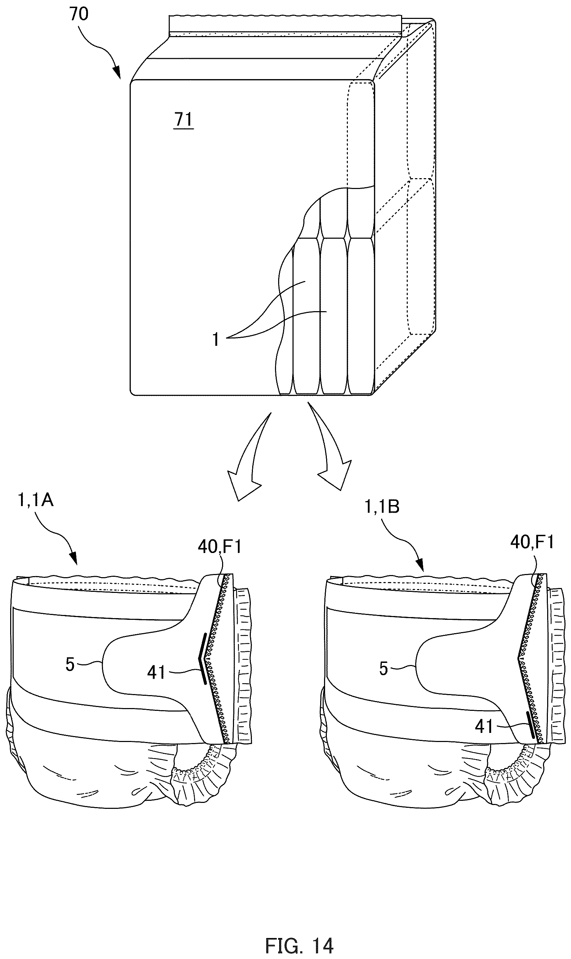

FIG. 14 is a diagram illustrating a package 70 of diapers 1. The package 70 of diapers 1 includes: a plurality of the aforementioned diapers 1; and a packaging member 71 that packages the plurality of diapers 1. It should be noted that, although the packaging member 71 illustrated in FIG. 14 has a rectangular-parallelepiped shape, the shape of the packaging member 71 is not particularly limited. For example, the packaging member 71 may be a packaging member such as a standing pouch, a flat bag, etc.

Among the plurality of diapers 1 packaged in the packaging member 71 illustrated in FIG. 14, a certain diaper 1A has a partially-cut portion 41 provided in the central portion, in the up-down direction, of the cutting-guide portion 40. Further, another diaper 1B packaged in the same packaging member 71 has a partially-cut portion 41 provided at the lower end portion of the cutting-guide portion 40.

As described above, even among diapers 1 packaged in the same packaging member 71, it is preferable that the relative position (the position in the up-down direction) of the partially-cut portion 41 relative to the cutting-guide portion 40 of a certain diaper 1A (first absorbent article) is different from the relative position (the position in the up-down direction) of the partially-cut portion 41 relative to the cutting-guide portion 40 of another diaper 1B (second absorbent article).

Since the position of the partially-cut portion 41 in the up-down direction differs among the diapers 1, a user using the plurality of diapers 1 can more readily recognize the overall position of the cutting-guide portion 40. Accordingly, the user can be more aware of cutting the waist portion 20, 30 along the cutting-guide portion 40, and can smoothly cut the waist portion 20, 30.

However, the above is not a limitation, and the relative position (the position in the up-down direction) of the partially-cut portion 41 relative to the cutting-guide portion 40 may be the same among the plurality of diapers 1 packaged in the packaging member 71.

### Other Embodiments:

Although embodiments of the present invention have been described hereinabove, the foregoing embodiments are to facilitate understanding of the present invention and are not to be construed as limiting the present invention. Further, it goes without saying that the present invention may variously be modified or altered without departing from its gist and encompasses equivalents thereof.

### REFERENCE SIGNS LIST

- 1:: Disposable diaper (absorbent article);
- 2a:: Side joining portion;
- 2b:: Side joining portion;
- 5:: Fastening portion;
- 5t:: Grip portion;
- 10:: Absorbent main body;
- 11:: Absorbent body;
- 11A:: Absorbent core;
- 12:: Top sheet;
- 13:: Second sheet;
- 14:: Side sheet;
- 15:: Back sheet;
- 16:: Exterior sheet;
- 17:: Leg stretchable/contractible member;
- 18:: Leak-proof-wall stretchable/contractible member;
- 20:: Front waist portion;
- 21:: Skin-side sheet;
- 22:: Non-skin-side sheet;
- 23:: Waist stretchable/contractible member;
- 25:: Engagement portion;
- 30:: Back waist portion;
- 31:: Skin-side sheet;
- 32:: Non-skin-side sheet;
- 33:: Waist stretchable/contractible member;
- 40:: Cutting-guide portion;
- 41:: Partially-cut portion;
- 42:: Non-cut portion;
- 43:: Non-cutting-guide portion;
- 44:: Welded portion;
- 51:: Engaging portion;
- 60:: Joining portion;
- 70:: Package of diapers 1 (absorbent article package);
- 71:: Packaging member;
- 80:: Crotch portion;
- BH:: Waist opening;
- LH:: Leg opening;
- F1:: Fixing portion;
- F2:: Auxiliary fixing portion (welded portion).

## Claims

1. An absorbent article having an up-down direction, a left-right direction, and a front-back direction intersecting with one another,
the absorbent article comprising:
an annular waist portion including a front waist portion and a back waist portion;
an absorbent main body; and
a fastening portion, a base end portion of the fastening portion being fixed to the waist portion by a fixing portion, and a leading end portion of the fastening portion including an engaging portion configured to detachably engage with a non-skin-side face of the waist portion,
the absorbent article including a cutting-guide portion configured to guide cutting of the waist portion in the up-down direction, and
the absorbent article including, in the cutting-guide portion, a partially-cut portion in which a portion of the waist portion in the up-down direction has been cut.

2. The absorbent article according to claim 1, wherein
at least a portion of the cutting-guide portion is located, in the left-right direction, between the fixing portion and an end of the engaging portion on the leading end portion side of the fastening portion, or
the cutting-guide portion is the fixing portion.

3. The absorbent article according to claim 2, wherein
in a stretched state of the waist portion, at least a portion of the partially-cut portion overlaps the engaging portion in the up-down direction.

4. The absorbent article according to claim 2, wherein
in a stretched state of the waist portion, the partially-cut portion does not overlap the engaging portion in the up-down direction.

5. The absorbent article according to any one of claims 2 to 4, wherein
at least a portion of the partially-cut portion overlaps the fastening portion in the front-back direction.

6. The absorbent article according to any one of claims 1 to 4, wherein
the waist portion includes, in the cutting-guide portion, a non-cut portion in which the waist portion has not been cut, and
in a stretched state of the waist portion, a length of the partially-cut portion in the up-down direction is different from a length of the non-cut portion in the up-down direction.

7. The absorbent article according to any one of claims 1 to 4, wherein
the waist portion is formed by a plurality of sheet members,
each of the plurality of sheet members is joined by an adhesive with the sheet member adjacent thereto in a thickness direction of the waist portion, and
the adhesive reaches at least an end of the partially-cut portion located on one side in the left-right direction.

8. The absorbent article according to any one of claims 1 to 4, wherein
the waist portion is formed by a plurality of sheet members,
each of the plurality of sheet members is joined by an adhesive with the sheet member adjacent thereto in a thickness direction of the waist portion, and
the adhesive is located so as to extend across the cutting-guide portion in the left-right direction.

9. The absorbent article according to any one of claims 1 to 4, wherein
the cutting-guide portion includes
an upper inclined portion that is inclined upwardly from a central portion in the up-down direction and toward the base end portion side of the fastening portion, and
a lower inclined portion that is inclined downwardly from the central portion and toward the base end portion side of the fastening portion, and
the partially-cut portion is located so as to extend across an intersection point between the upper inclined portion and the lower inclined portion.

10. The absorbent article according to any one of claims 1 to 4, wherein
the fastening portion includes a grip portion,
the grip portion is a portion of the fastening portion disposed further toward the leading end portion side than the engaging portion, and
in a stretched state of the waist portion, at least a portion of the partially-cut portion overlaps the grip portion in the up-down direction.

11. The absorbent article according to any one of claims 2 to 4, wherein
a color of a skin-side face of the fastening portion is different from a color of the non-skin-side face of the waist portion.

12. The absorbent article according to any one of claims 1 to 4, wherein
the waist portion is formed by one or more sheet members, and
a tensile strength of the one or more sheet members in the left-right direction is higher than a tensile strength of the one or more sheet members in the up-down direction.

13. The absorbent article according to any one of claims 1 to 4, wherein
the waist portion is formed by one or more sheet members,
the fastening portion is formed by one or more fastening-portion sheet members, and
a maximum value of a total grammage of the one or more sheet members is smaller than a minimum value of a total grammage of the one or more fastening-portion sheet members.

14. The absorbent article according to any one of claims 2 to 4, wherein
on a side further toward the base end portion of the fastening portion than the cutting-guide portion in the left-right direction, the absorbent article includes a joining portion that joins the fastening portion and the waist portion, and
in a stretched state of the waist portion, at least a portion of the partially-cut portion overlaps the joining portion in the up-down direction.

15. The absorbent article according to any one of claims 1 to 4, wherein
the partially-cut portion is located further inward than an end of the waist portion in the up-down direction, and
a plurality of the partially-cut portions are lined up in the up-down direction.

16. The absorbent article according to any one of claims 1 to 4, wherein
the absorbent article includes a non-cutting-guide portion at least on one side, in the up-down direction, of the cutting-guide portion.

17. The absorbent article according to claim 16, wherein
the absorbent article includes a welded portion in which the waist portion and the fastening portion are welded,
the welded portion is disposed adjacent to the cutting-guide portion on the base end portion side of the fastening portion in the left-right direction, and
in a stretched state of the waist portion, at least a portion of the non-cutting-guide portion overlaps the welded portion in the up-down direction.

18. An absorbent article package comprising:
a plurality of the absorbent articles according to claim 1; and
a packaging member that packages the plurality of absorbent articles,
the plurality of absorbent articles including a first absorbent article and a second absorbent article, and
a relative position of the partially-cut portion relative to the cutting-guide portion of the first absorbent article being different from a relative position of the partially-cut portion relative to the cutting-guide portion of the second absorbent article.
